# EUROPEAN PATENT APPLICATION

(11) **EP 4 623 935 A1**
(43) Date of publication of application: **01.10.2025**
(21) Application number: 23893624.9
(22) Date of filing: 07.11.2023
(51) Int. Cl.: A61K 47/64, C07K 19/00, C07K 1/20, C07K 1/18, A61K 38/27, A61P 3/00, A61P 17/02, A61P 5/06

(54) **CONJUGATE OF RECOMBINANT HUMAN SERUM ALBUMIN AND RECOMBINANT HUMAN GROWTH HORMONE AND PREPARATION METHOD THEREFOR**

(30) Priority: 24.11.2022 CN 202211496441
(71) Applicant: Wuhan Healthgen Biotechnology Corp, Wuhan, Hubei 430020 (CN)
(72) Inventor: YU, Wenhui, Wuhan, Hubei 430020 (CN); YANG, Daichang, Wuhan, Hubei 430020 (CN); DONG, Liangliang, Wuhan, Hubei 430020 (CN); CHEN, Rong, Wuhan, Hubei 430020 (CN); XIA, Jun, Wuhan, Hubei 430020 (CN)
(74) Representative: Plougmann Vingtoft a/s
(86) International application number: PCT/CN2023/130070
(87) International publication number: WO 2024/109533

(57) **Abstract**

A stable long-acting growth hormone that uses recombimant human serum albumin and recombinant humal growth hormone as ingredients and is formed by means of linker conjugation, as well as a preparation method therefor. An rHSA-hGHconjugate having a purity not lower than 95% is obtained after separation and purification.

## Description

### TECHNICAL FIELD

The present disclosure pertains to the field of bio-pharmaceuticals, and particularly relates to a conjugate formed from a recombinant human serum albumin and a recombinant human growth hormone, and a preparation method therefor.

### BACKGROUND

Natural human growth hormone is a protein secreted by the human pituitary gland, which is a single-chain peptide consisting of 191 amino acid residues with a molecular weight of 22 kDa. It is mainly used clinically for treating growth retardation in children and growth hormone deficiency in adults, promoting wound healing, and the like. Recombinant human growth hormone (rhGH) is obtained by recombining the human growth hormone gene into an expression vector by the genetic engineering method, and expressing a recombinant protein using different systems. The amino acid sequence and spatial conformation of rhGH are exactly the same as those of human growth hormone and have the same biological effects as human endogenous growth hormone. However, direct injection of rhGH results in a short half-life due to high clearance in the body, and the therapeutic effect can only be achieved when a certain plasma concentration is reached. In order to ensure the drug effect and simulate the secretion rhythm of growth hormone in the human body, patients need to receive injections daily. Meanwhile, the indications for growth hormone mostly involve diseases that require long-term medication, which greatly affects the medication compliance of the patients and increases the cost of medical care. In order to solve this problem, long-acting formulations have become a research hotspot in recent years.

Globally, there are currently three long-acting growth hormone products that have been approved for marketing. They are polyethylene glycol recombinant human somatropin injection (trade name: Jinsaizeng) from Changchun GeneScience Pharmaceuticals Co., Ltd. approved in 2014, which is also the only long-acting growth hormone formulation currently approved in China, Somapacitan developed by Novo Nordisk approved in the United States in 2020, and Skytrofa developed by Ascendis Pharma approved in the United States in 2021, respectively. On May 23, 2022, VISEN Pharmaceuticals announced that the domestic 3 phases of pivotal clinical trial of the long-acting growth hormone introduced thereby, Lonapegsomatropin (TransCon hGH for injection, Skytrofa), had reached the primary endpoint.

N-Succinimidyl 6-maleimidohexanoate (EMCS) is an amine-sulfydryl crosslinker containing an NHS ester and a maleimide reactive group, which are located at opposite ends of a medium-length spacer arm (9.4 angstroms). The NHS ester terminus is conjugated with a primary amine at pH 7-9 to form a stable amide bond; maleimide is reacted with -SH group at pH 6.5-7.5 to form a stable thioether bond. Based on this principle, after the conjugation reaction of the NHS ester terminus of DMSO-activated EMCS with the primary amine of rhGH, the maleimide of EMCS is reacted with the free -SH group of rHSA to form a stable rhHSA-rhGH conjugate product.

To date, there are only relevant studies on the fusion expression of rHSA and rhGH, and no relevant patent or literature reports on the *in vitro* conjugation of rHSA and rhGH have been found. In the present disclosure, rHSA is conjugated with rhGH *in vitro,* which overcomes the disadvantages of HSA fusion proteins, such as easy degradation, low expression level, and reduced biological activity, and has the characteristics of easy large-scale production, low cost, good stability, good biological activity and controllability, and the like.

### SUMMARY

An objective of the present disclosure is to provide a high-purity and stable long-acting growth hormone (a conjugate of human serum albumin and human growth hormone (recombinant human serum albumin (rHSA)-recombinant human growth hormone (rhGH))) obtained by conjugating rHSA and rhGH as raw materials via a linker group, and then subjecting the conjugate to separation and purification.

Another objective of the present disclosure is to provide a method for preparing the conjugate (rHSA-rhGH) described above.

Yet another objective of the present disclosure is to provide a pharmaceutical composition comprising the conjugate described above.

According to one aspect of the present disclosure, a conjugate of human serum albumin and human growth hormone is formed by conjugating a recombinant human serum albumin (rHSA) and a recombinant human growth hormone (rhGH) as raw materials via a linker group, wherein the linker group is N-succinimidyl 6-maleimidohexanoate (EMCS).

Preferably, the recombinant human serum albumin is a plant-derived recombinant human serum albumin (OsrHSA), and the recombinant human growth hormone is a plant-derived recombinant human growth hormone (OsrhGH).

According to another aspect of the present disclosure, in order to achieve the conjugation of the recombinant human serum albumin and the recombinant human growth hormone to form a stable conjugate, the present disclosure provides a method for preparing the conjugate of human serum albumin and human growth hormone, which comprises the following steps in sequence:
1) mixing a recombinant human growth hormone with activated N-succinimidyl 6-maleimidohexanoate to allow amino of the recombinant human growth hormone to be linked to an amino reactive group of the N-succinimidyl 6-maleimidohexanoate, so as to obtain a recombinant human growth hormone-EMCS intermediate conjugate product;
2) mixing the recombinant human growth hormone-EMCS intermediate conjugate product obtained in the step 1) with the recombinant human serum albumin to obtain a final conjugate product of human serum albumin and human growth hormone; and
3) purifying the obtained final conjugate product to obtain the conjugate of human serum albumin and human growth hormone.

Preferably, the method comprises the following steps:
a) mixing the recombinant human growth hormone with activated N-succinimidyl 6-maleimidohexanoate for a conjugation reaction, linking amino of the recombinant human growth hormone to the amino reactive group of the N-succinimidyl 6-maleimidohexanoate, and adding a stop buffer to stop the conjugation reaction after the conjugation is completed, wherein
   a concentration of the recombinant human growth hormone is 2-10 mg/mL,
   a concentration of the N-succinimidyl 6-maleimidohexanoate is 50-150 mmol/L,
   a molar ratio of the recombinant human growth hormone to the N-succinimidyl 6-maleimidohexanoate is 1: 1 to 1: 10,
   a buffer for the conjugation reaction is phosphate buffered saline at pH 6.8-7.4, and the stop buffer contains non-cysteine amino acids;
b) desalting the reaction product obtained in the step a) by a dextran gel chromatographic column to obtain an rhGH-EMCS intermediate conjugate product, wherein
   the dextran gel chromatographic column is selected from Bestdex G-25 C (coarse), Bestdex G-25 M (medium), Bestdex G-25 F (fine), and Bestdex G-25 SF (superfine);
   an equilibration buffer I for the chromatographic column is phosphate buffered saline at pH 6.8-7.4 containing 1.0-5.0 mM EDTA;
c) mixing the rhGH-EMCS intermediate conjugate product obtained in the step b) with the recombinant human serum albumin to obtain an rHSA-rhGH final conjugate product, wherein
   a molar ratio of the rhGH-EMCS intermediate conjugate product to the recombinant human serum albumin is 1:2 to 2:1;
   a final reaction concentration of the recombinant human serum albumin is 0.5-10 mg/mL;
   conditions for the conjugation are as follows: conjugation at room temperature for 1-2 h or at 2-8 °C for 3-4 h;
d) subjecting the final conjugate product obtained in the step c) to hydrophobic chromatography to obtain an rHSA-rhGH intermediate purified product, wherein
   a medium for the hydrophobic chromatography is selected from Phenyl Bestrose FF, Phenyl Bestrose HP, Butyl Bestrose FF, Octyl Bestrose 4FF, UniHR Phenyl 30L, and UniHR Butyl 30L; and
f) subjecting the rHSA-rhGH intermediate purified product obtained in the step d) to anion chromatography to obtain an rHSA-rhGH final purified product, wherein
   a medium for the anion chromatography is selected from Unigel 80Q and Nanogel 50Q.

More preferably, the method of the present disclosure comprises the following steps:
a) mixing 4-5 mg/mL recombinant human growth hormone with 100 mmol/L activated N-succinimidyl 6-maleimidohexanoate dissolved in DMSO for a conjugation reaction, wherein a molar ratio of the recombinant growth hormone to EMCS is 1:5, the conjugation reaction allows amino of the recombinant human growth hormone to be linked to an amino reactive group of the N-succinimidyl 6-maleimidohexanoate, the buffer for the conjugation reaction is phosphate buffered saline at pH 7.2, conditions for the conjugation reaction are as follows: conjugation at room temperature for 30 min to 1 h, and after the conjugation reaction is completed, glycine is added to stop the conjugation;
b) desalting the reaction product obtained in the step a) by a Bestdex G-25 M dextran gel chromatographic column to obtain the rhGH-EMCS intermediate conjugate product, wherein the equilibration buffer I is phosphate buffered saline at pH 7.2 containing 2.0 mM EDTA;
c) mixing the rhGH-EMCS intermediate conjugate product obtained in the step b) with a recombinant human serum albumin to obtain an rHSA-rhGH final conjugate product, wherein a molar ratio of the rhGH-EMCS to the recombinant human serum albumin is 1:1, a final reaction concentration of the recombinant human serum albumin is 0.8-1.2 mg/mL, and conditions for the reaction are as follows: conjugation at 2-8 °C for 3-4 h;
d) subjecting the rHSA-rhGH final conjugate product obtained in the step c) to Phenyl Bestrose HP hydrophobic chromatography to obtain an rHSA-rhGH intermediate purified product, comprising the following steps:
   d1) equilibrating a Phenyl Bestrose HP chromatographic column at a flow rate of 120-150 cm/h by using 3-5 CVs of an equilibration buffer II consisting of 10 mM phosphate buffer at pH 6.5 and 0.5 M ammonium sulfate;
   d2) adjusting the conductivity of the rHSA-rhGH final conjugate product with 3 M ammonium sulfate so that the conductivity of a sample of the rHSA-rhGH final conjugate product for loading is 75-78 mS/cm, adjusting the pH of the sample to 6.5, and loading all the sample at a flow rate of 120-150 cm/h;
   d3) eluting impurity protein by using 7 CVs of a washing buffer consisting of 10 mM phosphate buffer at pH 6.5 containing 1%-2% isopropanol and 0.3 M ammonium sulfate at a flow rate of 120-150 cm/h, wherein the conductivity of the washing buffer is 48-52 mS/cm; and
   d4) eluting the rHSA-rhGH intermediate purified product by using 5 CVs of an elution buffer I consisting of 10 mM phosphate buffer at pH 7.2 and 0.025 M ammonium sulfate at a flow rate of 120-150 cm/h, wherein the conductivity of the elution buffer I is 6-8 mS/cm, and collecting an rHSA-rhGH-enriched eluate to obtain the rHSA-rhGH intermediate purified product; and
e) subjecting the rHSA-rhGH intermediate purified product obtained in the step d) to Nanogel 50Q anion chromatography to obtain the rHSA-rhGH final purified product, comprising the following steps:
   e1) equilibrating a Nanogel 50Q chromatographic column at a flow rate of 340-360 cm/h with 5-10 CVs of an equilibration solution III consisting of 10 mM phosphate buffer at pH 7.0 and 120 mM sodium chloride;
   e2) dialyzing the rHSA-rhGH intermediate purified product 4-5 times against a dialysate consisting of 10 mM phosphate buffer at pH 7.0 and 120 mM sodium chloride, adjusting the pH of the dialyzed rHSA-rhGH intermediate purified product to 7.0, then filtering the dialyzed rHSA-rhGH intermediate purified product through a 0.22 µm filter membrane to obtain a loading solution, and loading the loading solution at a flow rate of 340-360 cm/h; and
   e3) eluting target protein at a flow rate of 340-360 cm/h by using 5 CVs of an elution buffer II consisting of 20 mM phosphate buffer at pH 6.7 and 160 mM sodium chloride, wherein the conductivity of the elution buffer II is 17-19 mS/cm, and collecting an rHSA-rhGH-enriched eluate to obtain the rHSA-rhGH final purified product, which is the conjugate of human serum albumin and human growth hormone.

According to yet another aspect of the present disclosure, a pharmaceutical composition comprising the conjugate of human serum albumin and human growth hormone described herein is provided. The conjugate of the present disclosure is combined with various pharmaceutically or physiologically acceptable auxiliary materials, excipients, and the like to form a pharmaceutical composition, which has the therapeutic effect of long-acting growth hormone.

The present disclosure provides a conjugate formed from a recombinant human serum albumin and a recombinant human growth hormone, wherein the conjugate has the effect of a stable long-acting growth hormone. The present disclosure also provides a preparation method therefor, wherein an rHSA-rhGH conjugate with a purity of not less than 95% can be obtained after preparation and separation and purification by the method of the present disclosure.

### BRIEF DESCRIPTION OF THE DRAWINGS

FIG. 1 shows the exploration of conjugation conditions for rhGH-EMCS, in which M represents the protein standard molecular weight Marker, and 0.5-8 mg/mL represents a conjugation concentration of rhGH.
FIG. 2 shows the exploration of conjugation conditions for rhGH-EMCS and rHSA, in which M represents the protein standard molecular weight Marker, and 0.5:1, 1:1, and 1:2 represent molar ratios of rhGH to rHSA.
FIG. 3 shows the screening of chromatographic media for primary purification, in which M represents the protein standard molecular weight Marker, load represents loading, FT represents flow through, W represents washing, and CIP represents cleaning in place; A shows the chromatography results on Phenyl Bestrose FF; B shows the chromatography results on Phenyl Bestrose HP.
FIG. 4 shows the Determination of chromatographic conditions for primary purification, in which M represents the protein standard molecular weight Marker, L represents loading, FT represents flow through, W represents washing, E represents elution, and CIP represents cleaning in place.
FIG. 5 shows the screening of chromatographic media for final purification, in which M represents the protein standard molecular weight Marker, load represents loading, FT represents flow through, W represents washing, and Elu represents elution; A shows the chromatographic results of Unigel 80Q; B shows the chromatographic results on Nanogel 50SP.
FIG. 6 shows chromatograms of the processes in three batches of small-scale trials.
FIG. 7 shows the changes in body weight, tail length, hepatosomatic index, and width of tibial epiphyseal plate of hypophysectomized rats after administration, in which compared with the model group, *P < 0.05, **P < 0.01, ***P < 0.001; compared with the rhGH standard group, ^{#}P < 0.05, ^{##}P < 0.01, ^{###}P < 0.001; compared with the OsrhGH positive drug group, ^{&}P < 0.05, ^{&&}P < 0.01, ^{&&&}P < 0.001.
FIG. 8 shows the electrophoretic detection result (A) of the conjugate product of OsrHSA and HSA with OsrhGH and the relative gray value (B) of the band of the target product (** indicates p < 0.01 compared with HSA).
FIG. 9 shows the determination results of the free sulfydryl content (A) and the sulfydryl freeness (molar concentration of free sulfydryl/molar concentration of protein) (B) of OsrHSA and HSA at the same concentration (50 mg/mL, 752 µmol/L) (** indicates p < 0.01 compared with HSA).

### DETAILED DESCRIPTION

The technical solutions of the present disclosure will be described in detail below by way of examples and drawings, so as to better illustrate the features and advantages of the present disclosure. The examples provided should be construed as illustrative of the methods of the present disclosure, and not in any way limiting the technical solutions disclosed herein.

### Material source:

Human serum albumin (pHSA or HSA): commercially available, Grifols, lot No. A3AFE01332;
recombinant human serum albumin expressed in rice seeds (OsrHSA): prepared according to patent Nos. CN100540667C and CN103880947A;
recombinant human growth hormone (OsrhGH, molecular weight: 22 kDa): prepared according to patent No. CN111057138A;
the other reagents and instruments used are all commercially available, unless otherwise stated.

### [Example 1] Determination of conjugation conditions for rHSA-rhGH

### 1. Determination of conjugation conditions for rhGH-EMCS

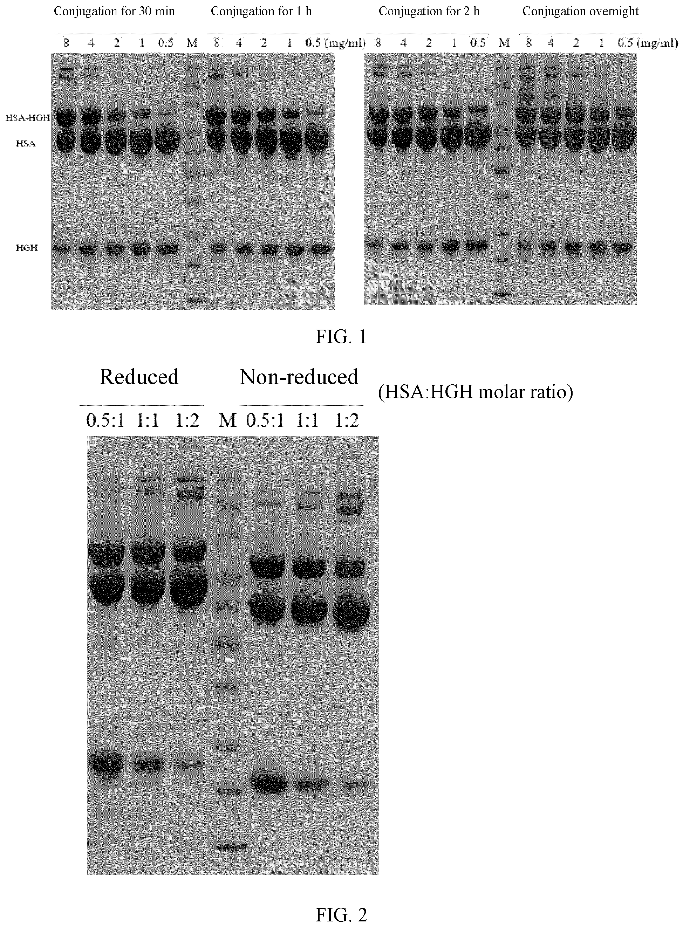

12 mL of each of 8 mg/mL, 4 mg/mL, 2 mg/mL, 1 mg/mL, and 0.5 mg/mL OsrhGH solutions was taken, and 0.3 mL of DMSO (about 2.5%) was added. Then, 218 µL, 109 µL, 55 µL, 28 µL, and 14 µL of EMCS solutions were each slowly added dropwise at a molar ratio of OsrhGH to EMCS (100 mmol/L) of 1:5. The mixture was stirred for a reaction at room temperature for 30 min, and then a 1 M Glycine solution with a molar amount 10 times that of EMCS was correspondingly added. After the reaction was completed, excessive EMCS was immediately removed using a Bestdex G-25 desalting column. 1310 µL, 655 µL, 328 µL, 162 µL, and 82 µL of OsrHSA (3 mmol/L) were each correspondingly added to the desalted OsrhGH-EMCS at a molar ratio of OsrHSA to OsrhGH-EMCS of 1:1. The mixture was reacted at room temperature for 30 min, 1 h, 2 h, and overnight, and then samples were taken for detection of conjugation (FIG. 1).

The results showed that: when conjugating different concentrations of OsrhGH, with the molar ratio of OsrhGH to EMCS fixed at 1:5, and the molar ratio of OsrhGH conjugated with EMCS to OsrHSA fixed at 1:1, the higher the concentration of OsrhGH, the more significantly the proportion of successful conjugation of OsrhGH-OsrHSA increased, and the conjugation success rate had a linear increasing trend when the concentration of OsrhGH was 0.5 mg/mL to 2 mg/mL; when the concentration of OsrhGH was greater than 2 mg/mL, the conjugation success rate increased (non-linearly), but the aggregate content also increased; with the increase of the reaction time, the proportion of successful conjugation increased, and the aggregate content also increased. Considering the instability of OsrhGH, the optimal conjugation concentration of OsrhGH was determined to be 4-5 mg/mL, and the conjugation time was determined to be 30 min to 1 h.

### 2. Determination of conjugation conditions for rhGH-EMCS and rHSA (recombinant human serum albumin)

14 mL of each of three 3.4 mg/mL OsrhGH solutions was taken, and 0.35 mL of DMSO (about 2.5%) was added. Then, 109 µL of EMCS solution was slowly added dropwise at a molar ratio of OsrhGH to EMCS of 1:5. The mixture was stirred for a reaction at room temperature for 30 min, and then 109 µL of 1 M Glycine solution with a molar amount 10 times that of EMCS was correspondingly added. After the reaction was completed, excessive EMCS was immediately removed using a Bestdex G-25 desalting column. 109 µL, 217 µL, and 434 µL of OsrHSA (3 mmol/L) were correspondingly added to the desalted OsrhGH-EMCS at molar ratios of OsrHSA to OsrhGH-EMCS of 0.5:1, 1:1, and 1:2, respectively. The mixture was reacted at room temperature for 1 h, and then samples were taken for detection of conjugation (FIG. 2).

The results showed that: when the molar ratio of OsrHSA to OsrhGH for conjugation was 0.5:1, the proportion of successful conjugation of OsrhGH-OsrHSA was the highest, and the aggregate content was the lowest, but the loss of OsrhGH was relatively large. Considering comprehensively, the molar ratio of OsrHSA to OsrhGH was preferably 1:1.

### [Example 2] Primary purification of rHSA-rhGH conjugate product

### 1. Selection of chromatographic medium for primary purification of conjugate product

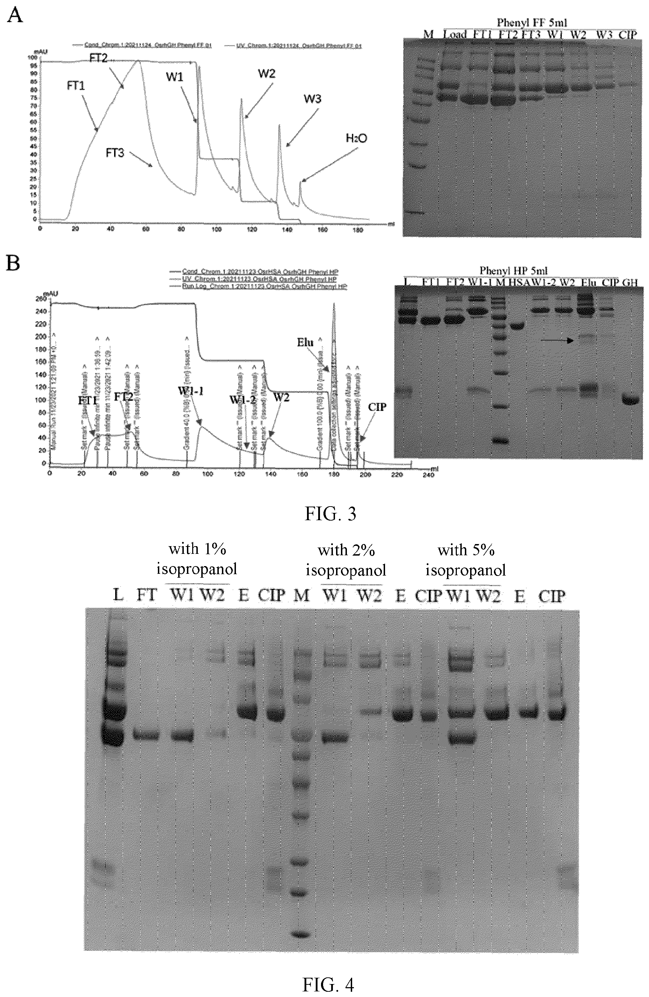

According to the optimal conjugation conditions determined in Example 1, a 5 mL Phenyl FF chromatographic column was equilibrated with 3-5 CVs of a solution consisting of 10 mM phosphate buffer at pH 7.2 and 0.15 M ammonium sulfate, and the conductivity of the OsrhGH-OsrHSA conjugate product was adjusted to be the same as that of the equilibration buffer with 3 M ammonium sulfate. The sample was loaded, then the column was washed in sequence with washing buffers consisting of 10 mM phosphate buffer at pH 7.2 containing 50 mM ammonium sulfate and 10 mM ammonium sulfate, respectively, and finally the product was eluted with 5 mM phosphate buffer (FIG. 3A). The results showed that: for the OsrHSA-OsrhGH conjugate product subjected to Phenyl FF chromatography, during sample loading, part of the conjugate product passed through the column while OsrHSA passed through the column.

According to the optimal conjugation conditions determined in Example 1, a 5 mL Phenyl Bestrose HP chromatographic column was equilibrated with 3-5 CVs of a solution consisting of 10 mM phosphate buffer at pH 7.2 and 0.5 M ammonium sulfate, and the conductivity of the OsrhGH-OsrHSA conjugate product was adjusted to be the same as that of the equilibration buffer with 3 M ammonium sulfate. The sample was loaded, then the column was washed in sequence with washing buffers consisting of 10 mM phosphate buffer at pH 7.2 containing 0.3 M ammonium sulfate and 0.2 mM ammonium sulfate, respectively, and finally the product was eluted with 10 mM phosphate buffer (FIG. 3B).

The results showed that: 95% of OsrHSA could be removed through Phenyl Bestrose HP chromatography. Therefore, Phenyl Bestrose HP could be preferably used as a chromatographic medium for primary purification.

### 2. Determination of primary purification conditions for conjugate product

According to the optimal conjugation conditions determined in Example 1, a 5 mL Phenyl Bestrose HP chromatographic column was equilibrated with 3-5 CVs of a solution consisting of 10 mM phosphate buffer at pH 6.5 and 0.5 M ammonium sulfate, and the conductivity of the OsrhGH-OsrHSA conjugate product was adjusted to be the same as that of the equilibration buffer with 3 M ammonium sulfate. The sample was loaded, then the column was washed in sequence with washing buffers consisting of 10 mM phosphate buffer at pH 6.5 containing 1%, 2%, and 5% isopropanol, respectively, and 0.22 M ammonium sulfate, and finally the product was eluted with a solution consisting of 10 mM phosphate buffer and 0.05 M ammonium sulfate (FIG. 4).

The results showed that: when the concentration of isopropanol in the washing buffer was increased to 1% and 2%, the content of unconjugated albumin in the final eluate was significantly reduced; when the washing was performed with 5% isopropanol, the target protein was eluted, indicating a relatively strong washing ability. Therefore, the concentration of isopropanol added was preliminarily determined to be 1%-2%.

### [Example 3] Final purification of rHSA-rhGH conjugate product

### 1. Selection of chromatographic medium for final purification of conjugate product

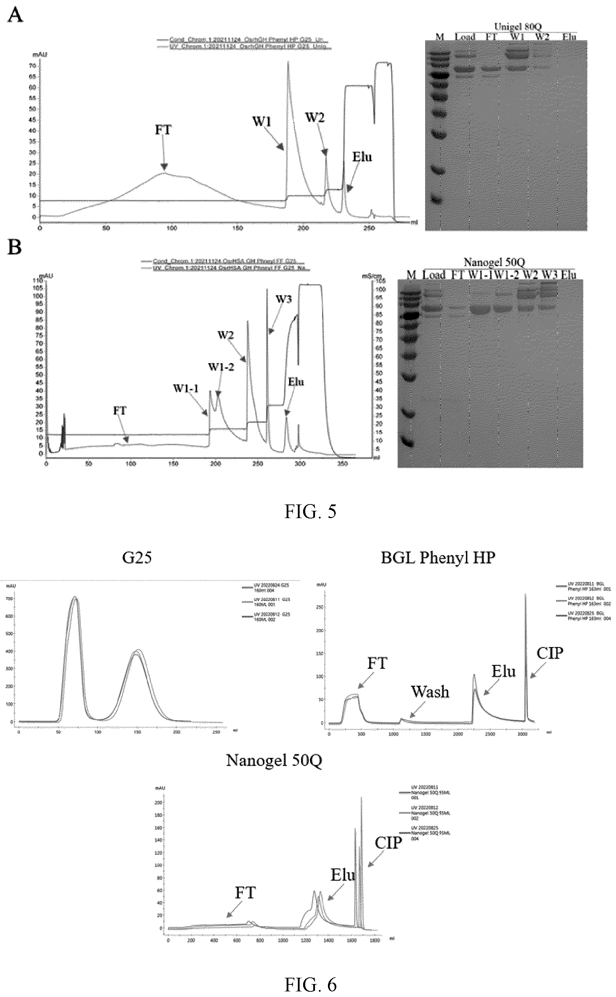

According to the optimal conjugation conditions and primary purification conditions determined in Examples 1-2, the screening of chromatographic media for final purification was performed. A 5 mL Unigel 80Q chromatographic column was equilibrated with 5-10 CVs of a solution consisting of 10 mM phosphate buffer at pH 7.2 and 0.12 M sodium chloride, and the OsrhGH-OsrHSA primary purified product was dialyzed against a dialysate consisting of 10 mM phosphate buffer at pH 7.2 and 0.12 M sodium chloride for 3-5 times, and then directly loaded. The column was washed in sequence with washing buffers consisting of 10 mM phosphate buffer at pH 7.2 containing 0.15 M sodium chloride and 0.2 M sodium chloride, respectively, and finally the product was eluted with a buffer consisting of 10 mM phosphate buffer at pH 7.2 and 1 M sodium chloride (FIG. 5A). A 5 mL Nanogel 50Q chromatographic column was equilibrated with 5-10 CVs of a solution consisting of 10 mM phosphate buffer at pH 7.2 and 0.12 M sodium chloride, and the OsrhGH-OsrHSA primary purified product was dialyzed against a dialysate consisting of 10 mM phosphate buffer at pH 7.2 and 0.12 M sodium chloride for 3-5 times, and then directly loaded. The column was washed in sequence with washing buffers consisting of 10 mM phosphate buffer at pH 7.2 containing 0.15 M sodium chloride, 0.2 M sodium chloride, and 0.3 M sodium chloride, respectively, and finally the product was eluted with a solution consisting of 10 mM phosphate buffer at pH 7.2 and 1 M sodium chloride (FIG. 5B).

The results showed that: under the same conditions, Nanogel 50Q had higher salt tolerance and better resolution. Therefore, Nanogel 50Q was subsequently selected as the chromatographic medium for final purification.

### 2. Determination of final purification conditions for conjugate product

16 mL of **4.8** mg/mL OsrhGH solution was taken. Then, 175 µL of EMCS solution was slowly added dropwise at a molar ratio of OsrhGH to EMCS of 1:5. The mixture was stirred for a reaction at room temperature for 30 min, and then a 1 M Glycine solution with a molar amount 10 times that of EMCS was correspondingly added. After the reaction was completed, excessive EMCS was immediately removed using a Bestdex G-25 desalting column. The desalted OsrhGH-EMCS (45 mL in total) was divided into 3 parts with 15 mL in each part. Each part was supplemented with 30 mL of PBS-EDTA at pH 7.2 to make a reaction volume of 45 mL. After the 2 parts were pre-cooled at 4 °C for 1 h, 364 µL of OsrHSA was added to each part at a molar ratio of OsrHSA to OsrhGH-EMCS of 1:1. The mixture was reacted at room temperature for 1 h for batch 001, at 4 °C for 1 h for batch 002, and at 4 °C for 4 h for batch 003. Chromatography was performed according to the primary purification conditions determined in Example 2 to obtain a primary purified product. A Nanogel 50Q chromatographic column was equilibrated with 5-10 CVs of a solution consisting of 10 mM phosphate buffer at pH 7.0 and 0.12 M sodium chloride. The OsrhGH-OsrHSA primary purified product was dialyzed against a dialysate consisting of 10 mM phosphate buffer at pH 7.0 and 0.12 M sodium chloride for 3-5 times, and then directly loaded. Finally, the product was eluted with a solution consisting of 20 mM phosphate buffer at pH 6.7 and 0.16 M sodium chloride.

The purities detected by SEC-HPLC are as follows:

| Lot. | Monomer (%) | Dimer (%) | Polymer (%) | Monomer + dimer (%) |
|---|---|---|---|---|
| Batch 001-B Elu | 93.45 | 3.69 | 2.86 | **97.14** |
| Batch 002-B Elu | 92.89 | 4.33 | 2.79 | **97.22** |
| Batch 003-B Elu | 93.73 | 4.24 | 2.04 | **97.97** |

The results showed that: for rhGH-EMCS conjugated with rHSA, by reducing the conjugation temperature to 2-8 °C and prolonging the conjugation time, with conjugation at room temperature, at 2-8 °C for 1 h, and at 2-8 °C for 4 h, SEC-HPLC showed that the purities of the final elution samples did not differ much (the purity of monomer was 92%-93%, and the purity of monomer + dimer was about 97%), while SDS-PAGE results showed that the purity was the highest after conjugation was performed at 2-8 °C for 4 h, and the yield was significantly increased from 14% to about 18% after conjugation was performed at 2-8 °C for 4 h. Therefore, a conjugation temperature of 2-8 °C and a conjugation time of 3-4 h are preferred for rhGH-EMCS and rHSA.

### [Example 4] Verification of conjugation and chromatography processes of three batches of small-scale trials of rHSA-rhGH

According to the optimal conjugation conditions, primary purification process, and final purification process determined in Examples 1-3, verification of the processes of three batches of small-scale trials was performed. The verification process and verification results are described below.

### 1. Conjugation process

A 4.5 mg/mL OsrhGH solution was taken, and an EMCS solution was slowly added dropwise at a molar ratio of OsrhGH to EMCS of 1:5. The mixture was stirred for a reaction at room temperature for 30 min, and then a 1 M Glycine solution with a molar amount 10 times that of EMCS was correspondingly added. After the reaction was completed, excessive EMCS was immediately removed using a Bestdex G-25 desalting column. The desalted OsrhGH-EMCS was supplemented with PBS-EDTA at pH 7.2 to make a volume 10 times the original volume, and OsrHSA was added at a molar ratio of OsrHSA to OsrhGH-EMCS of 1:1. The mixture was reacted at 4 °C for 3-4 h.

### 2. Chromatography process

A Phenyl Bestrose HP chromatographic column was equilibrated with 3-5 CVs of a solution consisting of 10 mM phosphate buffer at pH 6.5 and 0.5 M ammonium sulfate at a flow rate of 120-150 cm/h. The conductivity of the rHSA-rhGH conjugate product was adjusted with 3 M ammonium sulfate to achieve an optimal loading conductivity of 75-78 mS/cm, the pH was adjusted to 6.5, and the sample was completely loaded at a flow rate of 120-150 cm/h. The impurity proteins were eluted with a solution consisting of 10 mM phosphate buffer at pH 6.5 containing 1%-2% isopropanol and 0.3 M ammonium sulfate at a flow rate of 120-150 cm/h. The rHSA-rhGH conjugate product was eluted with a solution consisting of 10 mM phosphate buffer at pH 7.2 and 0.025 M ammonium sulfate at a flow rate of 120-150 cm/h, and an rHSA-rhGH-enriched eluate was collected to obtain an rHSA-rhGH intermediate purified product.

A Nanogel 50Q chromatographic column was equilibrated with 5-10 CVs of a solution consisting of 10 mM phosphate buffer at pH 7.0 and 120 mM sodium chloride at a flow rate of 340-360 cm/h. The intermediate purified product was dialyzed against a dialysate consisting of 10 mM phosphate buffer at pH 7.0 and 120 mM sodium chloride for 4-5 times, adjusted to pH 7.0, and filtered through a 0.22 µm filter membrane to obtain a loading solution. The loading solution was completely loaded at a flow rate of 340-360 cm/h. The target protein was eluted with a solution consisting of 20 mM phosphate buffer at pH 6.7 and 160 mM sodium chloride at a flow rate of 340-360 cm/h, and an rHSA-rhGH-enriched eluate was collected to obtain an rHSA-rhGH final purified product (FIG. 6).

The purities detected by SEC-HPLC are as follows:

| Sample name | Monomer (%) | Dimer (%) | Polymer (%) | HSA (%) | HGH (%) | Monomer + dimer (%) |
|---|---|---|---|---|---|---|
| Batch 001 B Elu | 93.70 | 4.41 | 0.38 | 0.36 | 1.14 | 98.11 |
| Batch 002 B Elu | 90.46 | 5.75 | 2.70 | 0.33 | 0.78 | 96.21 |
| Batch 004 B Elu | 91.24 | 7.19 | 0.52 | 0.09 | 0.95 | 98.43 |
| Batch 001 stock solution | 92.48 | 5.01 | 1.28 | 0.49 | 0.73 | 97.49 |
| Batch 002 stock solution | 89.54 | 5.66 | 3.83 | 0.44 | 0.54 | 95.20 |
| Batch 004 stock solution | 88.78 | 7.77 | 3.08 | 0.00 | 0.38 | 96.55 |

The results showed that: in the verification of the three batches of small-scale trials, the average yield was 15.5%, the RSD was 11.1%, and the sample purity by SEC-HPLC was greater than 95%. The verification of the three batches was qualified.

### [Example 5] Determination of efficacy of rHSA-rhGH in animals

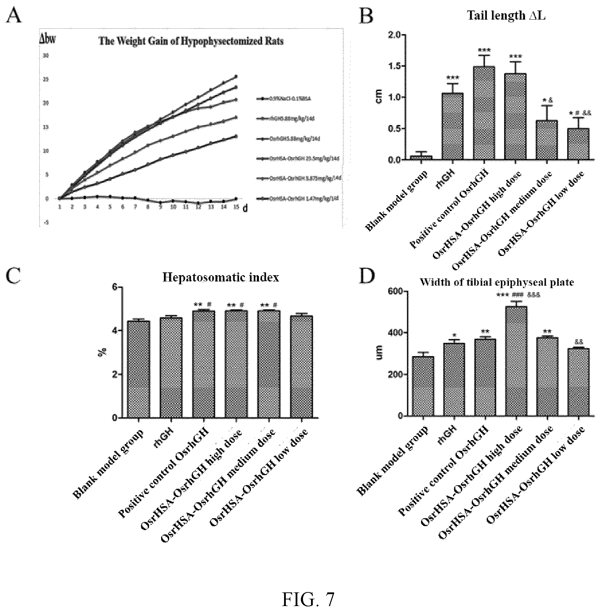

According to the optimal conjugation conditions, primary purification process, and final purification process determined in Examples 1-3, the samples for efficacy evaluation were prepared. The long-acting effect of the recombinant human serum albumin conjugated with recombinant human growth hormone was evaluated according to the growth-promoting effect on hypophysectomized juvenile SD rats, and the conjugate was compared with the short-acting OsrhGH and the national standard of recombinant human growth hormone (rhGH). Two to three weeks before the experiment, the pituitary glands of juvenile rats were surgically removed under clean conditions. Qualified and healthy hypophysectomized rats in which the weight change before the administration experiment was within ± 10% of the weight on the first day of the 2nd week after the operation were selected and randomly and evenly grouped according to the weight, with 8 rats in each group, for a total of 6 groups, including a test sample high-dose group, a test sample medium-dose group, a test sample low-dose group, an rhGH standard group, an OsrhGH positive drug group, and a model group. The administration dosages for the rhGH standard group and the positive control OsrhGH group were both 420 µg (1.26 IU)/kg/time/d, for 14 consecutive days, with a total dosage of 5.88 mg/kg/14d. The administration dosages for the test sample OsrHSA-OsrhGH high-dose, medium-dose, and low-dose groups were 23.5 mg/kg/14d, 5.875 mg/kg/14d, and 1.47 mg/kg/14d, respectively, for a total of 2 administrations, i.e., on d1 and d8. The model group was only subjected to administration with the vehicle, with the same administration frequency and volume as those of the test samples. On d15, after the rats were sacrificed, the growth indicators were measured, including body weight, tail length, liver weight, width of tibial epiphyseal plate, etc. The results showed that: within 15 days, the test sample OsrHSA-OsrhGH groups showed the same efficacy as the OsrhGH positive control group and the short-acting rhGH standard group, and significantly increased growth indicators of the hypophysectomized rats (FIG. 7). Compared with the model group, the body weight of the hypophysectomized rats in the test sample OsrHSA-OsrhGH high-dose, medium-dose, and low-dose groups is significantly increased (P < 0.01), showing a good growth curve, the tail length and the liver weight were significantly increased (P < 0.01), and the hepatosomatic index in groups other than the low-dose group was significantly increased (P < 0.01). Compared with the model group, the width of tibial epiphyseal plate of the test sample OsrHSA-OsrhGH high-dose and medium-dose groups was significantly increased (P < 0.01). The test sample OsrHSA-OsrhGH was administered once a week. The high-dose test sample, at a total dose equivalent to that of short-acting rhGH and OsrhGH, had the same pharmacodynamic effect as a conventional human growth hormone administered once daily for 14 consecutive days for replacement therapy, showing a long-acting effect. Conclusion: The test sample OsrHSA-OsrhGH has the pharmacodynamic effect of promoting the growth of organisms, and has a significant long-acting effect compared with the conventional human growth hormone administered daily.

### [Example 6] Comparison of conjugation efficiencies of plant-derived recombinant human serum albumin (OsrHSA) and blood-derived serum albumin (HSA) with recombinant human growth hormone

Recombinant human growth hormone (OsrhGH, molecular weight: 22 kDa) (prepared according to patent No. CN111057138A) was dissolved in phosphate buffered saline (PBS), and then an EMCS solution (100 mmol/L) was slowly added dropwise at a molar ratio of OsrhGH to EMCS of 1:5. The mixture was stirred for a reaction at room temperature for 30 min, and then a Glycine solution (1 mol/L) with a molar amount 5 times that of EMCS was added to stop the reaction.

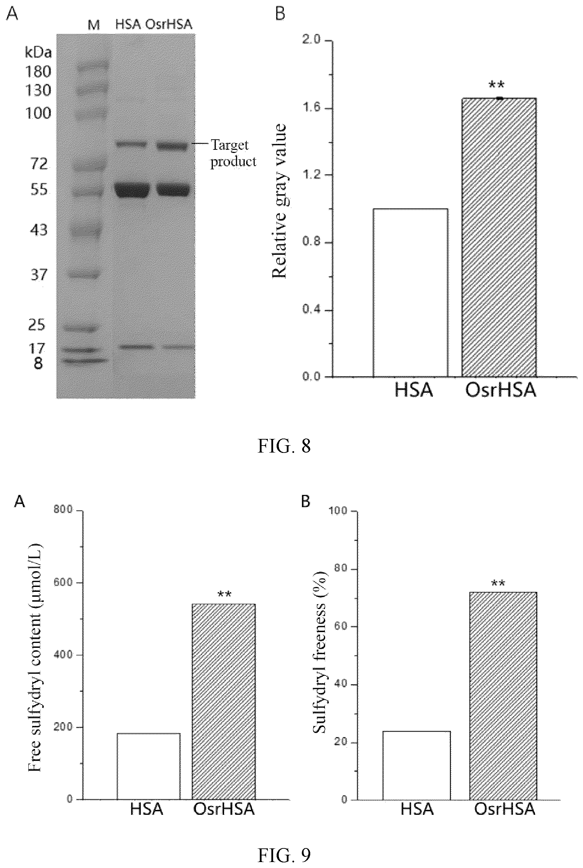

After the reaction was completed, excessive EMCS was removed using a Bestdex G-25 M desalting column. The desalted OsrhGH-EMCS was divided into 2 parts, and an OsrHSA injection and an HSA injection were added at a molar ratio of 1:1, respectively. The mixture was reacted at room temperature for 1 h. Then, with the same sample loading amount the conjugation efficiencies of OsrhGH with albumins from different sources were analyzed by 4%-20% SDS-PAGE. As can be seen from the electrophoresis results, the band gray value of the conjugate product (target product) in the OsrHSA reaction solution was significantly higher than that in the HSA reaction solution. According to the gray scale scanning calculation by Image J, the content of the target product in the OsrHSA reaction solution was about 1.66 times the content of the target product in the HSA reaction solution (FIG. 8).

### [Example 7] Determination of free sulfydryl content in OsrHSA and HSA

In order to illustrate the mechanism by which OsrHSA has a higher conjugation efficiency than HSA, the free sulfydryl content in OsrHSA and HSA was determined by using the DTNB method with cysteine serving as the basis for the standard curve. OsrHSA or HSA was diluted to 50 mg/mL (752 µmol/L) with a reaction buffer (100 mmol/L sodium phosphate, 1 mmol/L EDTA, pH 8.0) for determination. To a 5 mL reaction tube were added 2.5 mL of reaction buffer and 50 µL of DTNB reaction solution (4 mg/mL) in advance, and then 250 µL of cysteine standard solution (0.25-1.5 mmol/L) or the test sample was added. The mixture was reacted at room temperature for 15 min, and then the absorbance value at 412 nm was measured by a spectrophotometer. A standard curve was plotted according to the absorbance value of the cysteine standard solution, and then the sulfydryl content in the test sample was calculated. According to the calculation based on the standard curve, the free sulfydryl content in OsrHSA (752 µmol/L) was 541 µmol/L (the sulfydryl freeness was 72%), while the free sulfydryl content in HSA (752 µmol/L) was 183 µmol/L (the sulfydryl freeness was 24%). As can be seen from the determination results, the free sulfydryl content in OsrHSA is about 3 times that in HSA (FIG. 9).

## Claims

1. A conjugate of human serum albumin and human growth hormone, wherein the conjugate is formed by conjugating a recombinant human serum albumin and a recombinant human growth hormone as raw materials via a linker group, wherein the linker group is N-succinimidyl 6-maleimidohexanoate; the recombinant human serum albumin is a plant-derived recombinant human serum albumin.

2. The conjugate according to claim 1, wherein the recombinant human growth hormone is a plant-derived recombinant human growth hormone.

3. A method for preparing the conjugate of human serum albumin and human growth hormone according to claim 1, comprising the following steps in sequence:
1) mixing a recombinant human growth hormone with activated N-succinimidyl 6-maleimidohexanoate to allow an amino of the recombinant human growth hormone to be linked to an amino reactive group of the *N*-succinimidyl 6-maleimidohexanoate, so as to obtain a recombinant human growth hormone-EMCS intermediate conjugate product;
2) mixing the recombinant human growth hormone-EMCS intermediate conjugate product obtained in the step 1) with the plant-derived recombinant human serum albumin to obtain a human serum albumin-human growth hormone final conjugate product; and
3) purifying the obtained final conjugate product to obtain the conjugate of human serum albumin and human growth hormone.

4. The method according to claim 3, comprising the following steps:
4a) mixing the recombinant human growth hormone with activated N-succinimidyl 6-maleimidohexanoate for a conjugation reaction, linking the amino of the recombinant human growth hormone to the amino reactive group of the *N*-succinimidyl 6-maleimidohexanoate, and adding a stop buffer to stop the conjugation reaction after the conjugation is completed, wherein
a concentration of the recombinant human growth hormone is 2 mg/mL-10 mg/mL,
a concentration of the *N*-succinimidyl 6-maleimidohexanoate is 50 mmol/L-150 mmol/L,
a molar ratio of the recombinant human growth hormone to the *N*-succinimidyl 6-maleimidohexanoate is 1: 1 to 1: 10,
a buffer for the conjugation reaction is phosphate buffered saline at pH 6.8-7.4, and
the stop buffer contains non-cysteine amino acids;
4b) desalting the reaction product obtained in the step 4a) by a dextran gel chromatographic column to obtain the rhGH-EMCS intermediate conjugate product, wherein
the dextran gel chromatographic column is selected from Bestdex G-25 C (coarse), Bestdex G-25 M (medium), Bestdex G-25 F (fine), and Bestdex G-25 SF (superfine);
an equilibration buffer I for the chromatographic column is phosphate buffered saline at pH 6.8-7.4 containing 1.0 mM-5.0 mM EDTA;
4c) mixing the rhGH-EMCS intermediate conjugate product obtained in the step 4b) with the plant-derived recombinant human serum albumin to obtain the rHSA-rhGH final conjugate product, wherein
a molar ratio of the rhGH-EMCS intermediate conjugate product to the recombinant human serum albumin is 1:2 to 2:1;
a final reaction concentration of the recombinant human serum albumin is 0.5mg/mL-10 mg/mL;
conditions for the conjugation are as follows: conjugation at room temperature for 1 h-2 h or at 2°C-8 °C for 3 h-4 h;
4d) subjecting the rHSA-rhGH final conjugate product obtained in the step 4c) to hydrophobic chromatography to obtain an rHSA-rhGH intermediate purified product, wherein
a medium for the hydrophobic chromatography is selected from Phenyl Bestrose FF, Phenyl Bestrose HP, Butyl Bestrose FF, Octyl Bestrose 4FF, UniHR Phenyl 30L, and UniHR Butyl 30L; and
4f) subjecting the rHSA-rhGH intermediate purified product obtained in the step 4d) to anion chromatography to obtain an rHSA-rhGH final purified product, wherein
a medium for the anion chromatography is selected from Unigel 80Q and Nanogel 50Q.

5. The method according to claim 4, comprising the following steps:
5a) mixing 4-5 mg/mL recombinant human growth hormone with 100 mmol/L activated N-succinimidyl 6-maleimidohexanoate dissolved in DMSO for a conjugation reaction, wherein a molar ratio of the recombinant growth hormone to EMCS is 1:5, the conjugation reaction allows amino of the recombinant human growth hormone to be linked to an amino reactive group of the N-succinimidyl 6-maleimidohexanoate, the buffer for the conjugation reaction is phosphate buffered saline at pH 7.2, and conditions for the conjugation reaction are as follows: conjugation at room temperature for 30 min to 1 h, and after the conjugation reaction is completed, glycine is added to stop the conjugation;
5b) desalting the reaction product obtained in the step 5a) by a Bestdex G-25 M dextran gel chromatographic column to obtain the rhGH-EMCS intermediate conjugate product, wherein the equilibration buffer I is phosphate buffered saline at pH 7.2 containing 2.0 mM EDTA;
5c) mixing the rhGH-EMCS intermediate conjugate product obtained in the step 5b) with the plant-derived recombinant human serum albumin to obtain the rHSA-rhGH final conjugate product, wherein a molar ratio of the rhGH-EMCS to the plant-derived recombinant human serum albumin is 1:1, the final reaction concentration of the plant-derived recombinant human serum albumin is 0.8mg/mL-1.2 mg/mL, and conditions for the reaction are as follows: conjugation at 2°C-8 °C for 3h-4 h;
5d) subjecting the rHSA-rhGH final conjugate product obtained in the step 5c) to Phenyl Bestrose HP hydrophobic chromatography to obtain the rHSA-rhGH intermediate purified product, comprising the following steps:
d1) equilibrating a Phenyl Bestrose HP chromatographic column at a flow rate of 120-150 cm/h by using 3-5 CVs of 10 mM phosphate buffer at pH 6.5 and 0.5 M ammonium sulfate;
d2) adjusting the conductivity of the rHSA-rhGH final conjugate product with 3 M ammonium sulfate so that the conductivity of a sample of the rHSA-rhGH final conjugate product for loading is 75-78 mS/cm, adjusting the pH of the sample to 6.5 and loading all the sample at a flow rate of 120-150 cm/h;
d3) eluting impurity protein by using 7 CVs of a washing buffer consisting of 10 mM phosphate buffer at pH 6.5 containing 1%-2% isopropanol and 0.3 M ammonium sulfate at a flow rate of 120-150 cm/h, wherein the conductivity of the washing buffer is 48-52 mS/cm; and
d4) eluting the rHSA-rhGH intermediate purified product by using 5 CVs of an elution buffer I consisting of 10 mM phosphate buffer at pH 7.2 and 0.025 M ammonium sulfate at a flow rate of 120-150 cm/h, wherein the conductivity of the elution buffer I is 6-8 mS/cm, and collecting an rHSA-rhGH-enriched eluate to obtain the rHSA-rhGH intermediate purified product; and
5e) subjecting the rHSA-rhGH intermediate purified product obtained in the step 5d) to Nanogel 50Q anion chromatography to obtain the rHSA-rhGH final purified product, comprising the following steps:
e1) equilibrating a Nanogel 50Q chromatographic column at a flow rate of 340 cm/h-360 cm/h with 5CVs-10 CVs of an equilibration solution III consisting of 10 mM phosphate buffer at pH 7.0 and 120 mM sodium chloride;
e2) dialyzing the rHSA-rhGH intermediate purified product 4-5 times against a dialysate consisting of 10 mM phosphate buffer at pH 7.0 and 120 mM sodium chloride, adjusting the pH of the dialyzed rHSA-rhGH intermediate purified product to 7.0, then filtering the dialyzed rHSA-rhGH intermediate purified product through a 0.22 µm filter membrane to obtain a loading solution, and loading the loading solution at a flow rate of 340-360 cm/h; and
e3) eluting target protein at a flow rate of 340-360 cm/h by using 5 CVs of an elution buffer II consisting of 20 mM phosphate buffer at pH 6.7 and 160 mM sodium chloride, wherein the conductivity of the elution buffer II is 17 mS/cm-19 mS/cm, and collecting an rHSA-rhGH-enriched eluate to obtain the rHSA-rhGH final purified product, which is the conjugate of human serum albumin and human growth hormone.

6. The method according to any one of claims 3-5, wherein the recombinant human growth hormone is a plant-derived recombinant human growth hormone.

7. A pharmaceutical composition, comprising the conjugate of human serum albumin and human growth hormone according to claim 1.
